# EUROPEAN PATENT APPLICATION

(11) **EP 4 846 962 A1**
(43) Date of publication of application: **02.12.2026**
(21) Application number: 26181564.1
(22) Date of filing: 28.05.2026
(51) Int. Cl.: A61B 18/00

(54) **SKIN TREATMENT DEVICE AND METHOD FOR SETTING TREATMENT PARAMETER BASED ON HANDPIECE POSTITION**

(30) Priority: 30.05.2025 KR 20250071157
(71) Applicant: VIOL Medical Co., Ltd., Seongnam-si, Gyeonggi-do 13510 (KR)
(72) Inventor: CHO, Seunghwan, 13510 Seongnam-si, Gyeonggi-do (KR); JUNG, Mikyung, 13510 Seongnam-si, Gyeonggi-do (KR)
(74) Representative: Zimmermann, Tankred Klaus

(57) **Abstract**

The present disclosure relates to a skin treatment device, including a handpiece configured to be held by an operator, a position recognition sensor mounted on the handpiece and configured to communicate with a plurality of reference position sensors attached to a skin of a treatment subject, and a processor configured to recognize a treatment point position of the handpiece based on communication information between the position recognition sensor and the reference position sensors, and to set a treatment parameter related to a treatment operation of the handpiece based on the recognized treatment point position.

## Description

### BACKGROUND

### 1. Technical Field

The present disclosure relates to a skin treatment device and a method for setting a treatment parameter based on a handpiece position.

### 2. Related Art

In the field of skin cosmetic and therapeutic procedures, localized treatments using various energy-based devices (e.g., radio frequency, ultrasound, laser, etc.) are widely used. These devices are generally operated by bringing an operator's handpiece into contact with a specific area of the face or body to perform the procedure.

However, a conventional skin treatment device lacks the capability to accurately recognize or track the position of the handpiece, so that appropriate output settings or depth adjustments according to the treatment region are often performed manually. Thus, variations in treatment quality may occur depending on the operator's level of proficiency, and issues such as skin damage due to excessive output or reduced treatment efficacy due to insufficient output may arise.

In particular, when targeting complex curved surfaces such as the face, more precise position recognition and corresponding automated parameter adjustment are required because the thickness and sensitivity of the skin vary depending on the position. In addition, there is a lack of technical means for providing consistent output at the same position during repeated procedures.

Therefore, there is a growing need for a skin treatment device that can recognize the position of the handpiece in real time and automatically set parameters such as appropriate output, depth, and treatment time according to the treatment region, thereby simultaneously improving the precision, safety, and convenience of the procedure.

The description provided above as a related art of the present disclosure is for helping understand the background of the present disclosure and may include information that does not constitute prior art.

### (Prior Art Document)

### (Patent Documents)

(Patent Document 1) Korean Patent No. 10-2751776 (January 3, 2025)

### SUMMARY

The present disclosure has been devised to solve the above problems, and an object of the present disclosure is to provide a skin treatment device and a method for setting a treatment parameter based on a handpiece position, which can recognize a treatment point position of a handpiece on a skin region such as a face and set a treatment parameter related to a treatment operation of the handpiece based on the recognized position.

A skin treatment device according to an embodiment of the present disclosure may include a handpiece configured to be held by an operator, a position recognition sensor mounted on the handpiece and configured to communicate with a plurality of reference position sensors attached to a skin of a treatment subject, and a processor configured to recognize a treatment point position of the handpiece based on communication information between the position recognition sensor and the reference position sensors, and to set a treatment parameter related to a treatment operation of the handpiece based on the recognized treatment point position.

A method for setting a treatment parameter of a skin treatment device according to an embodiment of the present disclosure may include acquiring, by the processor, reference position information from a plurality of reference position sensors attached to a skin of a treatment subject through the position recognition sensor, recognizing, by the processor, a treatment point position of the handpiece using the reference position information, and setting, by the processor, a treatment parameter related to the treatment operation of the handpiece based on the recognized treatment point position.

According to an embodiment of the present disclosure, a treatment point position of a handpiece can be precisely recognized on a three-dimensional coordinate system using a plurality of reference position sensors attached to the skin (e.g., a facial area) of a treatment subject and a position recognition sensor mounted on the handpiece.

According to an embodiment of the present disclosure, a treatment parameter related to a treatment operation of a handpiece may be set based on a treatment point position of the handpiece.

According to an embodiment of the present disclosure, parameter information for a skin region corresponding to a treatment point position of a handpiece may be generated using parameter information according to skin conditions stored in a memory and skin condition information for each skin region input from a skin measurement device, and a treatment parameter may be set based on the generated parameter information.

According to an embodiment of the present disclosure, by recognizing a treatment point position of a handpiece and automatically setting a treatment parameter, it is possible to perform procedures with consistent quality regardless of an operator's level of proficiency or experience, and to simultaneously improve the safety and effectiveness of the procedure by applying a parameter optimized for characteristics of each region.

According to an embodiment of the present disclosure, since a parameter is automatically set without manual operation by an operator, it is possible to achieve reduced treatment time and improved user convenience.

According to an embodiment of the present disclosure, by linking a treatment region with past treatment history data, it is possible to prevent duplicate treatments and enable extensions such as AI-based region-specific treatment prediction and personalized treatment settings, thereby providing a technical basis for implementing an intelligent treatment system.

### BRIEF DESCRIPTION OF THE DRAWINGS

FIGS. 1 and 2 are block diagrams illustrating a skin treatment device according to an embodiment of the present disclosure.
FIG. 3 is a view illustrating an example in which a plurality of reference position sensors are attached to a face, in an embodiment of the present disclosure.
FIG. 4 is a view illustrating another example in which a plurality of reference position sensors are attached to a face, in an embodiment of the present disclosure.
FIG. 5 is a view exemplarily illustrating a usage state of the skin treatment device according to an embodiment of the present disclosure, limited to a face.
FIG. 6A is a view illustrating a process of generating parameter information for each skin region according to an embodiment of the present disclosure.
FIG. 6B is a view exemplarily illustrating information in a first table and a second table of FIG. 6A.
FIG. 7 is a flowchart illustrating a method for setting a treatment parameter based on a handpiece position of a skin treatment device according to an embodiment of the present disclosure.
FIG. 8 is a flowchart illustrating a method for re-recognizing a treatment point position of a handpiece according to a posture change of a treatment subject in an embodiment of the present disclosure.

### DETAILED DESCRIPTION

Hereinafter, preferred embodiments of the present disclosure will be described in detail with reference to the accompanying drawings.

The embodiments are provided to more fully explain the present disclosure to those skilled in the art, and the following embodiments may be modified in various other forms, and the scope of the present disclosure is not limited to the following embodiments. Rather, these embodiments are provided to make this disclosure more thorough and complete, and to fully convey the concept of the present disclosure.

The terminology used herein is for the purpose of describing particular embodiments and is not intended to limit the present disclosure. In addition, as used herein, the singular forms are intended to include the plural forms as well, unless the context clearly indicates otherwise. As used herein, terms such as "include," "comprise," and "have" are intended to specify the presence of stated features, numbers, steps, operations, components, parts, or combinations thereof, and should be understood as not precluding the presence or addition of one or more other features, numbers, steps, operations, components, parts, or combinations thereof.

The drawings are provided only to facilitate understanding of the concept of the present disclosure and should not be construed as limiting the scope of the present disclosure. Further, in the drawings, relative thicknesses, lengths, or relative sizes may be exaggerated for convenience and clarity of description.

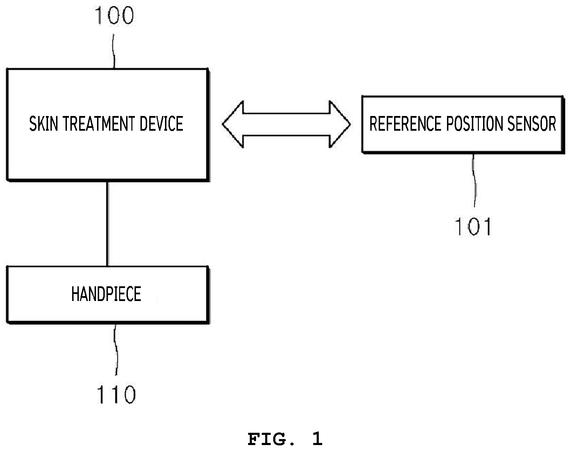

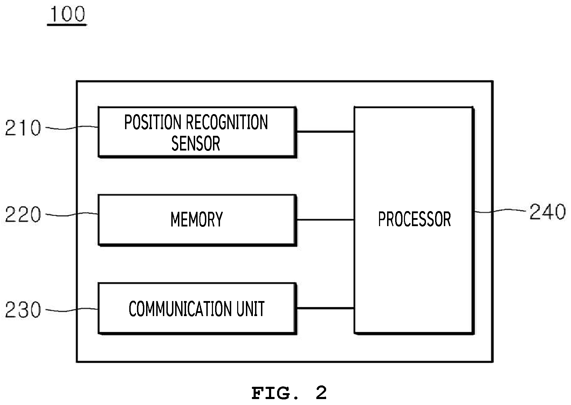

FIGS. 1 and 2 are block diagrams illustrating a skin treatment device according to an embodiment of the present disclosure.

Referring to FIGS. 1 and 2, the skin treatment device 100 according to an embodiment of the present disclosure may include a handpiece 110, a position recognition sensor 210, a memory 220, a communication unit 230, and a processor 240.

The handpiece 110 is configured to be held by an operator and directly manipulated on a skin treatment region, and is equipped with at least some of the components 210, 220, 230, and 240 required for the operation of the skin treatment device 100. According to an embodiment, the skin treatment device 100 may be configured as an all-in-one handpiece type. In the case of the all-in-one handpiece type, all of the components 210, 220, 230, and 240 of the skin treatment device 100 are accommodated within the handpiece 110 to form a single device. Alternatively, the skin treatment device 100 may be configured as a split type in which the handpiece 110 and a main console (not shown) are separated. In the case of the split type, the handpiece 110 is communicatively connected to the main console, which is a main body of the skin treatment device 100, via a wired or wireless connection, and some of the components may be mounted in the main console.

The position recognition sensor 210 may be mounted on the handpiece 110. The position recognition sensor 210 may be configured to communicate with a plurality of reference position sensors 101. In this case, the plurality of reference position sensors 101 may be attached to the skin of a treatment subject. Specifically, an operator brings the handpiece 110 to the skin of the treatment subject, that is, a treatment skin region, and performs a skin-invasive procedure. When the handpiece 110 approaches the treatment skin region, the position recognition sensor 210 mounted on the handpiece 110 communicates with the plurality of reference position sensors 101. Here, the skin to which the plurality of reference position sensors 101 are attached may be, for example, the face of the treatment subject, but is not limited thereto.

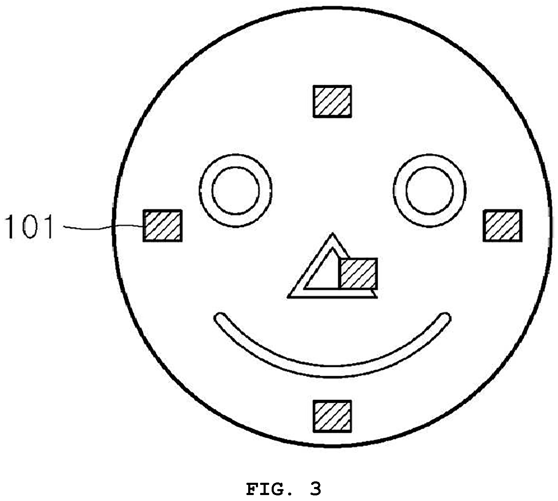

FIG. 3 is a view illustrating an example in which the plurality of reference position sensors 101 are attached to the face in an embodiment of the present disclosure. As shown in FIG. 3, the plurality of reference position sensors 101 may be attached to respective regions of the face at predetermined intervals. For example, the plurality of reference position sensors 101 may be attached to a center of the forehead, a tip of the chin, a cheekbone protrusion, and beside a nostril.

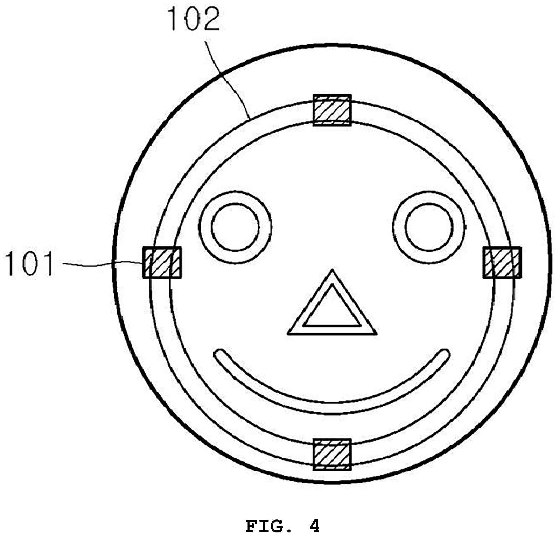

In this case, the plurality of reference position sensors 101 may be attached by various methods such as a skin adhesive type, a wearable mount structure, a skin insertion type or protrusion attachment type, a magnetic attachment type, and a suction type. The skin adhesive type is a method in which a medical-grade skin adhesive is applied to a rear surface of the reference position sensor 101 to directly attach it to the skin (e.g., a disposable sensor patch, an ECG electrode). The wearable mount structure is a method in which a structure incorporating the reference position sensors 101 is worn on the face and the reference position sensors 101 are positioned at specific regions, which will be described in detail with reference to FIG. 4. The skin insertion type or protrusion attachment type is a method of slightly fixing the reference position sensor 101 to the skin or minimizing movement using fine protrusions. The magnetic attachment type is a method in which a magnetic plate or a metal plate is attached near the surface of the face, and the reference position sensor 101 is fixed by magnetic force. The suction type is a method of adhering to a curved surface of the face using a gel or silicone suction pad.

Meanwhile, the plurality of reference position sensors 101 may be attached to three or more selected facial regions among facial regions including the center of the forehead, the tip of the chin, the cheekbone protrusion, and beside the nostril. Here, the reason for attaching three or more reference position sensors 101 to the facial regions is that the face has a three-dimensional structure. That is, since the face has the three-dimensional structure, at least three reference position sensors 101 need to be attached to the face so as to accurately recognize a treatment position of the handpiece 110. In other words, in order to recognize a treatment point position of the handpiece 110 on the three-dimensional face, it is necessary to generate a reference coordinate system, and the treatment point position of the handpiece 110 may be relatively calculated based on this reference coordinate system. This will be described in detail later.

FIG. 4 is a view illustrating another example in which the plurality of reference position sensors 101 are attached to the face in an embodiment of the present disclosure, and relates to the above-described wearable mount structure. As shown in FIG. 4, the plurality of reference position sensors 101 may be attached to a structure 102 disposed to surround the face of a treatment subject, and may be spaced apart from each another. According to an embodiment, the reference position sensors 101 may be attached one by one on the structure 102 in east, west, south, and north directions. For example, the reference position sensors 101 may be respectively attached to the forehead, the tip of the chin, and both cheekbone regions among facial regions, and as described above, at least three sensors need to be attached.

In this case, the reference position sensors 101 may be fixedly attached to the structure 102 or may be detachably attached. The structure 102 is preferably stably fixed without movement when placed on the face of the treatment subject. This is because, if the structure 102 shakes or changes position, a reference coordinate system (to be described later) based on the reference position sensors 101 may change, which may cause errors in recognizing the treatment point position of the handpiece 110. To this end, the structure 102 may be formed in a circular shape; however, the shape is not limited thereto, and any shape may be used as long as it can be stably fixed on the face without movement.

Turning back to FIGS. 1 and 2, the memory 220 may store a first table in which parameter information according to skin conditions is matched. Further, the memory 220 may store a second table in which treatment history data for each treatment subject is matched. Of course, the memory 220 may store a plurality of instructions and data required for execution by the processor 240, and may store data processed by the processor 240.

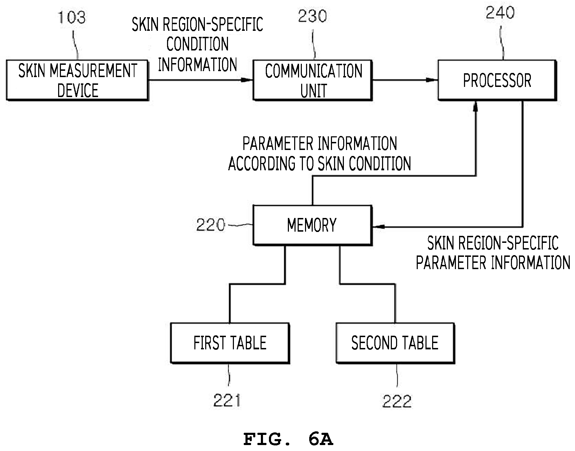

The communication unit 230 may perform wired or wireless communication with other devices or networks. To this end, the communication unit 230 may include a communication module that supports at least one of various wired and wireless communication methods. For example, the communication module may be implemented in the form of a chipset. The communication unit 230 may communicate with an external skin measurement ( 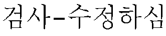 , 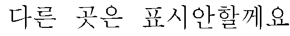 ) device 103 (see FIG. 6A) through the communication module, and thereby obtain skin condition information for each skin region from the external skin measurement device. This will be described in detail with reference to FIG. 6A.

The processor 240 may recognize the treatment point position of the handpiece 110 based on communication information between the position recognition sensor 210 and the reference position sensors 101. According to an embodiment, the processor 240 may, through the position recognition sensor 210, read reference position information corresponding to each of pre-stored positions of the reference position sensors 101, calculate distances between each of the reference position sensors 101 and the position recognition sensor 210 using the read reference position information, and recognize the treatment point position of the handpiece 110 based on the calculated distances and the reference position information.

To this end, the processor 240 may generate the reference coordinate system based on the skin region to which the reference position sensors 101 are attached, using the reference position information. The processor 240 may recognize the treatment point position of the handpiece 110 by applying the calculated distances and the reference position information to the reference coordinate system. That is, the treatment point position of the handpiece 110 may be calculated based on the reference coordinate system.

Meanwhile, the treatment subject may not maintain the same posture during the treatment operation of the handpiece 110 and may move, and the treatment point position of the handpiece 110 may change according to such posture changes of the treatment subject. In this case, the treatment region of the handpiece 110 may change, so that a problem may arise in that the treatment parameter also needs to be changed. To address this, it is necessary to re-recognize the treatment point position of the handpiece 110 according to the posture change of the treatment subject.

To this end, the processor 240 may detect a change in the treatment point position of the handpiece 110 according to the posture change of the treatment subject based on reference position information acquired during the treatment operation of the handpiece 110, and may calculate a variation of the reference coordinate system corresponding to the change in the treatment point position. The processor 240 may update the reference coordinate system based on the calculated variation, and may re-recognize the treatment point position of the handpiece 110 using the updated reference coordinate system.

The processor 240 may set treatment parameters related to the treatment operation of the handpiece 110 based on the treatment point position of the handpiece 110. Here, the treatment parameters may include energy output intensity, treatment depth, spot size, number of treatments, treatment time, and speed.

According to an embodiment, the processor 240 may store, in the memory 220, skin region-specific condition information indicating the condition of the skin region of the treatment subject and parameter information according to skin conditions, and then may set treatment parameters based on the skin region-specific condition information, the parameter information according to skin conditions stored in the memory 220, and the treatment point position of the handpiece 110. That is, the processor 240 may generate parameter information for each skin region using the skin region-specific condition information and the parameter information according to skin conditions stored in the memory 220, and may recognize a corresponding skin region based on the treatment point position of the handpiece 110, thereby setting treatment parameters for the corresponding skin region.

According to another embodiment, the processor 240 may store, in the memory 220, the first table in which parameter information according to skin conditions is matched, and when skin region-specific condition information is input from an external source, may generate parameter information for each skin region by referring to the first table, and recognize a corresponding skin region based on the treatment point position of the handpiece 110, thereby setting treatment parameters for the corresponding skin region. In this case, the processor 240 may obtain the skin region-specific condition information from the external skin measurement device 103 (see FIG. 6A) through the communication unit 230.

The processor 240 may control the treatment operation of the handpiece 110 using the set treatment parameters. For example, the processor 240 may control treatment operations such as output energy magnitude, irradiation depth, output time, and number of shots of the handpiece 110 using the set treatment parameters.

Meanwhile, the processor 240 may store, in the memory 220, the second table in which treatment history data for each treatment subject is matched.

Here, the treatment history data may include basic information such as a unique identifier (ID), date of birth, gender, skin type, medical history information, and medication history of the treatment subject. In addition, the treatment history data may include treatment date and time information, a treatment target region (e.g., forehead, cheekbone, chin, etc.), a type and model name of a used treatment device, a type and specification of a used handpiece or cartridge, applied treatment parameters (e.g., output intensity, treatment depth, frequency, number of shots, shot density, treatment mode, pulse interval, etc.), and operator information (e.g., ID of medical personnel or operator). Furthermore, the treatment history data may also include treatment result information such as post-treatment reaction information (e.g., erythema, swelling, pain, etc.), comparative photographs taken before and after the treatment, a subjective satisfaction level of the treatment subject, a recovery period, and whether side effects have occurred (e.g., pain, burns, bruising, etc.).

The processor 240 may generate a learning model based on treatment history by using matching information of the second table stored in the memory 220. When skin region-specific condition information is input from the external source, the processor 240 may generate optimized parameter information for each skin region in a personalized manner based on the learning model, and may set treatment parameters related to the treatment operation of the handpiece 110 based on the generated parameter information for each skin region.

That is, the processor 240 may generate, for each treatment subject, the learning model based on the treatment history by using matching information between each treatment subject and the corresponding treatment history data. When skin region-specific condition information of a particular treatment subject is input from the external source, the processor 240 may apply the input skin region-specific condition information to the learning model to generate, in a personalized manner, parameter information for each skin region optimized for the particular treatment subject, and may set treatment parameters optimized in the personalized manner based on the generated parameter information for each skin region.

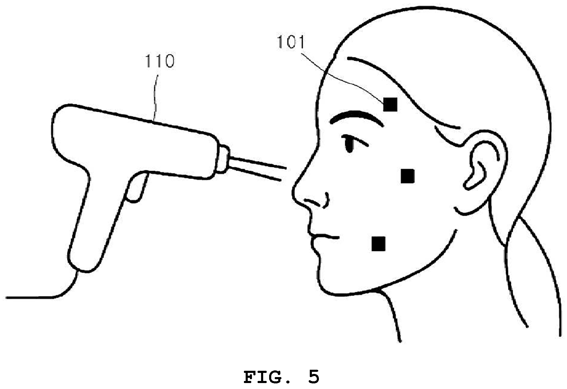

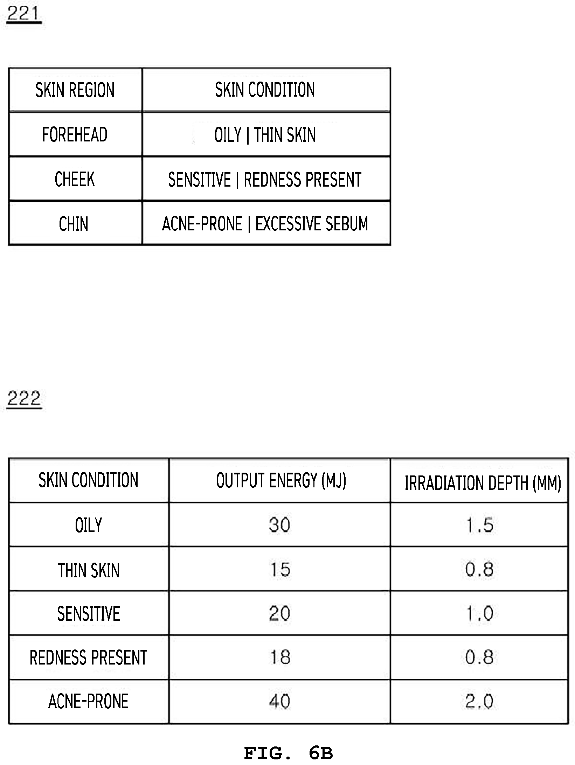

FIG. 5 is a view exemplarily illustrating the usage state of the skin treatment device according to an embodiment of the present disclosure, limited to a face, FIG. 6A is a view illustrating the process of generating the parameter information for each skin region according to an embodiment of the present disclosure, and FIG. 6B is a view exemplarily illustrating information in the first table and the second table of FIG. 6A.

Referring to FIG. 5, when the operator intends to perform the treatment on the face of the treatment subject using the handpiece 110, it is necessary to recognize the treatment point position of the handpiece 110. To this end, in an embodiment of the present disclosure, reference position information corresponding to positions of each of the reference position sensors 101 attached to the face of the treatment subject may be read through the position recognition sensor 210 (see FIG. 2) mounted on the handpiece 110, distances between each of the reference position sensors 101 and the position recognition sensor may be calculated using the read reference position information, and the treatment point position of the handpiece 110 may be recognized based on the calculated distances and the reference position information.

In this case, the reference coordinate system based on the face of the treatment subject may be generated, and the reference coordinate system may serve as a basis for calculating the treatment point position of the handpiece 110. That is, according to an embodiment of the present disclosure, the facial region where the handpiece 110 is located may be recognized by calculating the treatment point position of the handpiece 110 based on distances between each of the reference position sensors 101 and the position recognition sensor and the reference position information, with respect to the reference coordinate system. When the position of the handpiece 110 is recognized in this manner, a process of setting treatment parameters related to the treatment operation of the handpiece 110 may be performed.

In this regard, a more detailed description will be given with reference to FIGS. 6A and 6B. The processor 240 may store, in the memory 220, the first table 221 in which parameter information according to skin conditions is matched. The processor 240 may obtain skin region-specific condition information from the external skin measurement device 103 through the communication unit 230, load parameter information according to skin conditions from the first table 221 of the memory 220 based on the skin region-specific condition information, generate parameter information for each skin region, and store the generated parameter information in the memory 220. This series of processes may be performed before the position of the handpiece 110 is recognized.

After the position of the handpiece 110 is recognized, the processor 240 may determine a corresponding skin region based on the treatment point position of the handpiece 110. The processor 240 may load parameter information matched to the corresponding skin region from the memory 220, and may set treatment parameters related to the treatment operation of the handpiece 110 based on the loaded parameter information.

According to an embodiment, the first table 221 is a database in which specific regions of skin to be treated (e.g., forehead, cheek, chin, etc.) are mapped with types of skin conditions (e.g., oily, sensitive, acne-prone, etc.) frequently observed in the corresponding regions. This is configured in consideration of physiological characteristics in which skin properties vary depending on the treatment region. For example, the forehead has a high density of sebaceous glands, resulting in a higher prevalence of oily skin, whereas the cheek area tends to frequently exhibit sensitivity reactions or redness due to external irritation. Such skin region-specific condition information may be based on results measured by the skin measurement device 103, but is not limited thereto and may also be based on prior expert clinical analysis or direct user selection.

According to an embodiment, the second table 222 is a database in which treatment parameters (e.g., output energy, irradiation depth) optimized for each skin condition are mapped based on the skin conditions derived from the first table 221. The output energy represents an intensity of energy delivered to the skin per unit irradiation and is carefully adjusted according to the skin condition. For example, regions with excessive sebum secretion or severe acne may require relatively stronger energy, whereas sensitive or thin skin should be treated with lower energy to minimize irritation. The irradiation depth refers to a depth to which laser or radio frequency energy penetrates into the skin, and is appropriately adjusted depending on a position of a lesion or tissue thickness. These treatment parameters are stored in the device based on predefined clinical outcome-based criteria, and may be automatically set according to a skin condition selected by a user, or may be designed to allow manual adjustment when necessary.

For example, when a user selects a chin region, skin conditions such as acne or excessive sebum may be automatically mapped from the first table 221, and corresponding output energy (e.g., 40 mJ) and irradiation depth (e.g., 2.0 mm) may be extracted through the second table 222 and set as treatment parameters. Thereby, the operator can perform a stable and precise procedure with settings optimized for the skin condition without separately adjusting the treatment parameters.

Meanwhile, the processor 240 may store the second table 222 in which treatment history data for each treatment subject is matched. The second table 222 is intended to provide personalized treatment parameters optimized for a specific treatment subject. The processor 240 may generate the learning model based on treatment history using matching information of the second table 222. Thereafter, when skin region-specific condition information is input from the external skin measurement device 103, the processor 240 may apply the input skin region-specific condition information to the learning model to generate optimized parameter information for each skin region in a personalized manner, and may store the generated parameter information in the memory 220. This series of processes may be performed before the position of the handpiece 110 is recognized.

After the position of the handpiece 110 is recognized, the processor 240 may determine a corresponding skin region based on the treatment point position of the handpiece 110. The processor 240 may load parameter information of a specific treatment subject matched to the corresponding skin region from the memory 220, and may set treatment parameters related to the treatment operation of the handpiece 110 in a personalized manner optimized for the specific treatment subject based on the loaded parameter information.

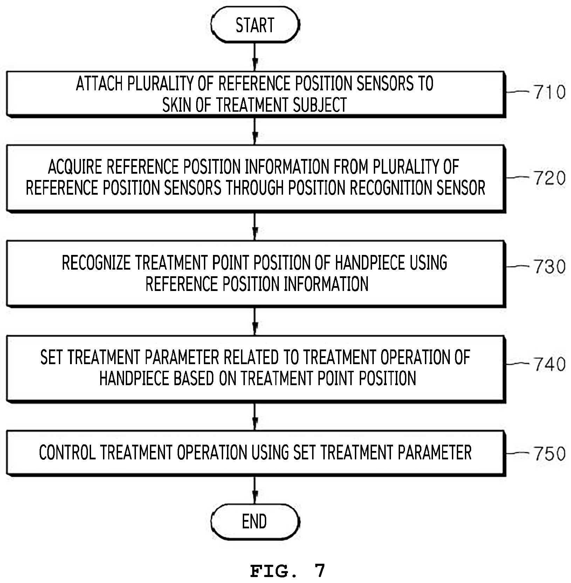

FIG. 7 is a flowchart illustrating a method for setting a treatment parameter based on a handpiece position of a skin treatment device according to an embodiment of the present disclosure.

Referring to FIGS. 1, 2, and 7, in step 710, the plurality of reference position sensors 101 may be attached to the skin of the treatment subject.

In this case, the reference position sensors 101 may be attached to the plurality of facial regions selected from among the center of the forehead, the tip of the chin, the cheekbone protrusion, and beside the nostril (see FIG. 3). Alternatively, the reference position sensors 101 may be attached on the circular structure arranged to surround the face of the treatment subject while being spaced apart from each other (see FIG. 4).

Next, in step 720, the processor 240 may acquire reference position information from the plurality of reference position sensors attached to the skin of the treatment subject through the position recognition sensor 210.

Next, in step 730, the processor 240 may recognize the treatment point position of the handpiece 110 using the reference position information.

Next, in step 740, the processor 240 may set treatment parameters related to a treatment operation of the handpiece 110 based on the treatment point position of the handpiece 110.

Next, in step 750, the processor 240 may control the treatment operation using the set treatment parameters.

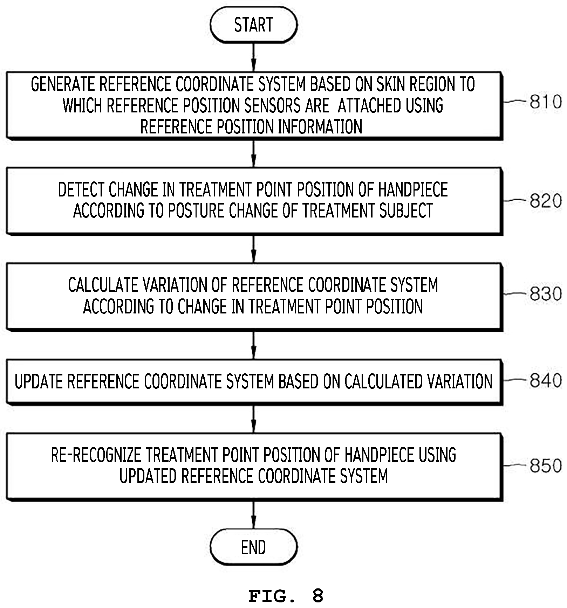

FIG. 8 is a flowchart illustrating a method for re-recognizing a treatment point position of a handpiece according to a posture change of a treatment subject in an embodiment of the present disclosure.

Referring to FIGS. 1, 2, and 8, in step 810, the processor 240 may generate the reference coordinate system based on the skin region to which the reference position sensors 101 are attached, using reference position information corresponding to positions of each of the reference position sensors 101.

Next, in step 820, the processor 240 may detect a change in the treatment point position of the handpiece 110 according to the posture change of the treatment subject based on reference position information acquired during the treatment operation of the handpiece 110.

Next, in step 830, the processor 240 may calculate the variation of the reference coordinate system according to a change in the treatment point position.

Next, in step 840, the processor 240 may update the reference coordinate system based on the calculated variation.

Next, in step 850, the processor 240 may re-recognize the treatment point position of the handpiece 110 using the updated reference coordinate system.

The devices (units) described above may be implemented by hardware elements and/or software elements. For example, the hardware elements may include a microphone, an amplifier, a band-pass filter, an A/D converter, and a processing device. The processing device may be implemented using one or more general-purpose computers or special-purpose computers, such as a processor, a controller, an arithmetic logic unit (ALU), a digital signal processor, a microcomputer, a field programmable gate array (FPGA), a programmable logic unit (PLU), a microprocessor, or other devices capable of responding to and executing instructions in a defined manner. The processing device may operate an operating system (OS) and one or more software applications running on the operating system. Further, the processing device may access, store, manipulate, process, and generate data in response to execution of the software. For convenience of description, the processing device may be described as a single device; however, those skilled in the art will understand that the processing device may include a plurality of processing elements and/or multiple types of processing elements. For example, the processing device may include a plurality of processors or a processor and a controller. In addition, other processing configurations, such as a parallel processor, are also possible.

The software may include a computer program, code, instructions, or a combination thereof, and may independently or collectively configure the processing device or instruct the processing device to operate as desired. The software and data may be embodied, either permanently or temporarily, in a propagated signal wave that may be interpreted by the processing device or that may provide instructions or data to the processing device, or in various types of machines, components, physical devices, virtual equipment, computer storage media, or devices. The software may be distributed over computer systems connected through a network, and may be stored and executed in a distributed manner. The software and data may be stored in one or more computer-readable recording media, which include data storage devices storing data and allowing the data to be read later by the computer system or the processing device. A method according to an embodiment may be implemented in the form of program instructions that may be executed by various computer means and recorded on the computer-readable medium. Examples of the computer-readable recording media include ROM, RAM, CD-ROM, magnetic tape, floppy disk, and optical data storage devices. Examples include magnetic media such as hard disks, floppy disks, and magnetic tapes; optical media such as CD-ROMs and DVDs; magneto-optical media such as floptical disks; and hardware devices specially configured to store and execute program instructions, such as ROM, RAM, and flash memory. In addition, functional programs, code, and code segments for carrying out the examples disclosed herein can be easily understood and implemented by a programmer having ordinary skill in the art to which these examples pertain, based on or using the descriptions provided herein in conjunction with the flowcharts and block diagrams of the drawings.

Although various embodiments have been described above, it should be understood that various modifications can be made. For example, even if the described techniques are performed in a different order, and/or the elements of the described systems, structures, devices, circuits, and the like are combined in a different manner, or are replaced or supplemented by other elements or equivalents, appropriate results may still be achieved. Accordingly, other implementations also fall within the scope of the following claims.

## Claims

1. A skin treatment device, comprising:
a handpiece configured to be held by an operator;
a position recognition sensor mounted on the handpiece and configured to communicate with a plurality of reference position sensors attached to a skin of a treatment subject; and
a processor configured to recognize a treatment point position of the handpiece based on communication information between the position recognition sensor and the reference position sensors, and to set a treatment parameter related to a treatment operation of the handpiece based on the recognized treatment point position.

2. The skin treatment device of claim 1, wherein the processor
reads, through the position recognition sensor, reference position information corresponding to a position of each of pre-stored reference position sensors,
calculates a distance between each of the reference position sensors and the position recognition sensor using the read reference position information, and
recognizes the treatment point position of the handpiece based on the calculated distance and the reference position information.

3. The skin treatment device of claim 2,
wherein the processor generates a reference coordinate system based on a skin region to which the reference position sensors are attached using the reference position information, and
wherein the treatment point position of the handpiece is calculated based on the reference coordinate system.

4. The skin treatment device of claim 3, wherein the processor
detects a change in the treatment point position of the handpiece according to a posture change of the treatment subject based on reference position information acquired during the treatment operation of the handpiece,
calculates a variation of the reference coordinate system according to the change in the treatment point position,
updates the reference coordinate system based on the calculated variation, and
re-recognizes the treatment point position of the handpiece using the updated reference coordinate system.

5. The skin treatment device of claim 1, further comprising:
a memory configured to store skin region-specific condition information indicating a condition of the skin region of the treatment subject and parameter information according to skin conditions,
wherein the processor sets the treatment parameter based on the skin region-specific condition information, the parameter information according to skin conditions stored in the memory, and the treatment point position.

6. The skin treatment device of claim 1, further comprising:
a memory configured to store a first table in which parameter information according to skin conditions is matched,
wherein the processor generates parameter information for each skin region by referring to the first table when skin region-specific condition information is input from an external source.

7. The skin treatment device of claim 6, further comprising:
a communication unit configured to communicate with an external skin measurement device,
wherein the processor acquires the skin region-specific condition information from the external skin measurement device through the communication unit.

8. The skin treatment device of claim 6, wherein the processor controls the treatment operation including at least one of output energy magnitude, irradiation depth, output time, and shot count of the handpiece using the set treatment parameter.

9. The skin treatment device of claim 1, further comprising:
a memory configured to store a second table in which treatment history data for each treatment subject is matched,
wherein the processor
generates a learning model based on treatment history using matching information of the second table,
generates optimized parameter information for each skin region in a personalized manner based on the learning model when skin region-specific condition information is input from the external source, and
sets the treatment parameter based on the generated parameter information for each skin region.

10. A method for setting a treatment parameter based on a handpiece position of a skin treatment device comprising a handpiece equipped with a position recognition sensor and a processor controlling a treatment operation of the handpiece, the method comprising:
acquiring, by the processor, reference position information from a plurality of reference position sensors attached to a skin of a treatment subject through the position recognition sensor;
recognizing, by the processor, a treatment point position of the handpiece using the reference position information; and
setting, by the processor, a treatment parameter related to the treatment operation of the handpiece based on the recognized treatment point position.

11. The method of claim 10, further comprising:
attaching the plurality of reference position sensors to the skin of the treatment subject,
wherein the attaching comprises attaching the reference position sensors to a plurality of facial regions selected from among a center of the forehead, a tip of the chin, a cheekbone protrusion, and beside a nostril.

12. The method of claim 10, further comprising:
attaching the plurality of reference position sensors to the skin of the treatment subject,
wherein, in the attaching, the reference position sensors are attached on a circular structure arranged to surround the face of the treatment subject while being spaced apart from each other.
